# EUROPÄISCHE PATENTSCHRIFT

(11) **EP 0 829 298 B1**
(45) Veröffentlichungstag und Bekanntmachung des Hinweises auf die Patenterteilung: **03.07.2002**
(21) Anmeldenummer: 97115933.0
(22) Anmeldetag: 12.09.1997
(51) Int. Cl.: B01J 2/00, B01J 2/02, B01J 2/26

(54) **Verfahren zur Herstellung von Hydroxipivalinsäureneopentylglykolester-Granulaten**
Method for making hydroxy pivalic acid neopentyl glycol ester granulates
Procédé de fabrication de granulats de l'ester néopentylglycol de l'acide hydroxypivalique

(30) Priorität: 13.09.1996 DE 19637380
(43) Veröffentlichungstag der Anmeldung: 18.03.1998
(73) Patentinhaber: BASF AKTIENGESELLSCHAFT, 67056 Ludwigshafen (DE)
(72) Erfinder: Weyer, Hans-Jürgen, Dr., 67240 Bodenheim-Roxheim (DE); Eck, Bernd, 68519 Viernheim (DE); Baumann, Dieter, 67227 Frankenthal (DE); Maltry, Bernhard, 67283 Obrigheim (DE)
(74) Vertreter: Isenbruck, Günter, Dr.

(56) Entgegenhaltungen:
- EP-A- 0 210 415
- DE-A- 3 209 747
- DE-A- 4 445 880
- US-A- 2 881 230

## Beschreibung

Die Erfindung betrifft ein Verfahren zur Herstellung von Hydroxipivalinsäureneopentylglykolester-Granulaten, die erhaltenen Granulate und die Verwendung einer Granuliervorrichtung zur Herstellung der Granulate.

Hydroxipivalinsäureneopentylglykolester (HPN) wird unter anderem als Bestandteil von Innenbeschichtungen von Konservendosen verwendet, um die Schlagzähigkeit der Innenbeschichtungen zu erhöhen. Zur Konfektionierung von HPN wird üblicherweise eine HPN-Schmelze über einen Tropfenformer auf ein Kühlband aufgebracht. Dabei entstehen flache schuppenförmige Granulate, die bei der Lagerung in Gebinden zum Verbacken neigen.

Das Verbacken erschwert dabei die Dosierung und Entnahme der Granulate aus den Gebinden.

Aus der DE-A-35 22 359 ist ein Verfahren zum Konfektionieren von kristallinen organischen Materialien, wie Hydroxipivalinsäureneopentylglykolester, bekannt, wobei ein Produkt mit verbesserter Rieselfähigkeit erhalten wird. Dabei wird in einer zweiwelligen Schneckenmaschine mit im gleichen Sinne rotierenden Schneckenwellen ein pulverförmiges Material verdichtet oder ein schmelzflüssiges Material bei Massetemperaturen, die etwa 1 bis 20°C unterhalb des Schmelzpunktes des eingesetzten Materials liegen, kristallisiert und verdichtet. Das Material wird sodann durch eine beheizte Lochplatte in eine Zone mit niedrigerem Druck ausgestoßen. Die Lochplatte wird auf eine Temperatur von 1 bis 30°C oberhalb des Schmelzpunktes des Materials erwärmt, so daß die an der Wandung der Lochplatte vorbeigeführten Einzelkristalle zu einem Film aufschmelzen, welcher nach seiner Erstarrung ein festes Korsett für das kompaktierte kristalline Material bildet. Die extrudierten Materialstränge werden in einer nachfolgenden Zone zerkleinert und gekühlt. Die Rieselfähigkeit der so erhaltenen Granulate ist nicht für alle Anwendungen ausreichend.

Aus der DE-C-32 09 747 ist eine Vorrichtung zur Herstellung von Granulat aus einer Schmelze bekannt, bei der ein zweiphasiges Gemisch aus einer Schmelze, die Kristallkeime enthält, mit einem Rotortropfenformer auf ein Kühlband gegeben wird. Es werden dabei halbkugelförmige Granulate erhalten.

Aufgabe der vorliegenden Erfindung ist die Bereitstellung eines Verfahrens zur Herstellung von HPN-Granulaten, das zu verbackungsarmen Granulaten führt und die Nachteile bekannter Verfahren vermeidet.

Diese Aufgabe wird erfindungsgemäß gelöst durch ein Verfahren zur Herstellung von Hydroxipivalinsäureneopentylglykolester-Granulaten durch Aufbringen einer Hydroxipivalinsäureneopentylglykolester-Schmelze auf eine Kühlfläche, vorzugsweise ein Kühlband, auf der/dem die Schmelze erstarrt, das dadurch gekennzeichnet ist, daß die Schmelze 15 bis 55 Gew.-%, bezogen auf die Gesamtmenge an Hydroxypivalinsäureneopentylglykolester, an Hydroxypivalinsäureneopentylglykolester-Kristallen enthält die durch Führen einer Hydroxipivalinsäureneopentylglykolester-Schmelze durch ein Vorkristallisator erzeugt werden, wobei die Hydroxipivalinsäureneopentylglykolester-Schmelze zunächst durch einen Vorkühler und dann durch den Vorkristallisator geführt wird hinter dem ein Teil der Schmelze abgezweigt und in den Vorkristallisator zurückgeführt wird und der verbleibende Teil der Schmelze über einen Tropfenformer oder ein Flächenaufgabesystem auf ein kontinuierlich angetriebenes Kühlband aufgebracht wird und auf diesem erstarrt und bei Verwendung eines Flächenaufgabesystems nach Verlassen des Kühlbandes in einem Brecher in Granulatform gebracht wird.

Mit Hilfe des erfindungsgemäßen Verfahrens ist es möglich, HPN-Granulate herzustellen, die in Gebinden keine Neigung zu Verbackungen zeigen, da die Kontaktflächen zwischen einzelnen Granulatteilchen klein sind. Insbesondere die im wesentlichen sphärischen HPN-Granulate, die durch tropfenweises Aufbringen der Schmelze erhalten werden, sind sehr verbackungsarm.

Vorzugsweise enthält die Schmelze vor dem Abkühlen 30 bis 50 Gew.-%, bezogen auf die Gesamtmenge an HPN, an HPN-Kristallen. Die HPN-Kristalle beziehungsweise -Kristallkeime werden in der HPN-Schmelze in einem Vorkristallisator erzeugt, in dem die Schmelze mechanisch bewegt und gleichzeitig gekühlt wird. Die Schmelze wird dazu in einem Vorkühler auf eine Temperatur nahe des Schmelzpunktes abgekühlt. Für HPN liegt diese Temperatur in der Nähe von 50°C. Der Durchlauf durch den Vorkühler und den Vorkristallisator kann so eingestellt werden, daß der gewünschte Gehalt an Kristallen in der Schmelze am Ausgang des Vorkristallisators vorliegt. Es wird ein Teil der aus dem Vorkristallisator austretenden Schmelze in den Vorkristallisator zurückgeführt, d.h. zwischen Vorkühler und Vorkristallisator wieder eingespeist.

Dabei ist es durch Einstellung des Rücklaufverhältnisses hinter dem Vorkristallisator und Wahl einer geeigneten Durchflußmenge und Temperatur im Vorkristallisator möglich, einen gewünschten Anteil an Kristallen in der Schmelze zu erzeugen.

Die Erfindung wird nachstehend weiter anhand der Zeichnung erläutert, in der

Figur 1 eine Vorrichtung für die Herstellung von HPN-Granulaten zeigt, in der
1 HPN-Schmelze
2 Vorkühler
3 Vorkristallisator
4 Tropfenformer
6 Kühlband
bedeutet.

Die Vorrichtung dient zur Herstellung von tropfenförmigen Granulaten.

Figur 2 zeigt eine Vorrichtung zur Herstellung von isometrischen oder quaderähnlichen Granulaten, wobei
1 HPN-Schmelze
2 Vorkühler
3 Vorkristallisator
5 Flächenaufgabesystem
6 Kühlband
7 Brecher
bedeutet.

Die erfindunggemäß verwendeten Vorrichtungen sind an sich bekannt. Eine Vorrichtung mit einem Tropfenformer ist beispielsweise in der DE-C-32 09 747 beschrieben.

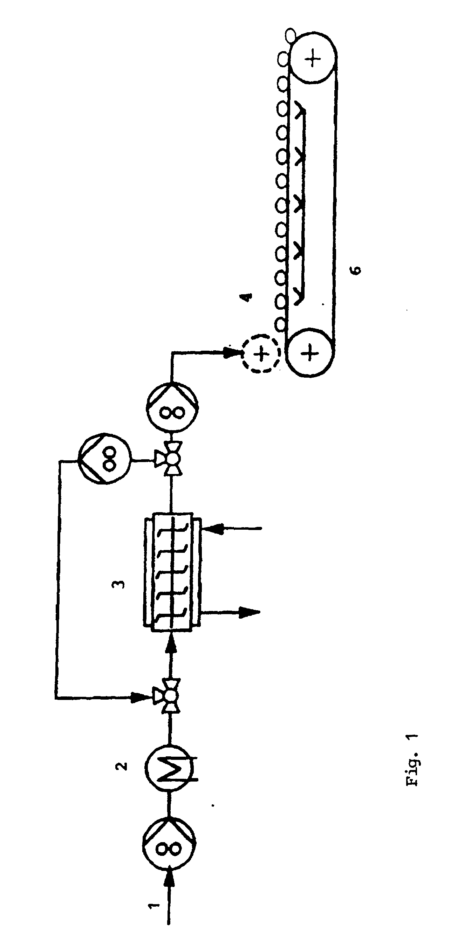

Gemäß der in Figur 1 abgebildeten Ausführungform wird die HPN-Schmelze (1) zunächst durch einen Vorkühler (2) und dann durch den Vorkristallisator (3) geführt, hinter dem ein Teil der Schmelze abgezweigt und in den Vorkristallisator (3) zurückgeführt wird. Der verbleibende Teil der Schmelze wird über einen Tropfenformer (4) auf ein kontinuierlich angetriebenes Kühlband (6) aufgebracht und erstarrt auf diesem. Am Ende des Kühlbandes können die Granulatteilchen entnommen werden. Aufgrund des hohen Gehalts an Kristallen in der Schmelze weist diese eine hohe Viskosität auf und erstarrt schnell. Dadurch ist es möglich, im wesentlichen sphärische HPN-Granulate zu erzeugen, die vorzugsweise einen mittleren Teilchendurchmesser von 3 bis 16, besonders bevorzugt 4 bis 10, insbesondere 5 bis 7 mm aufweisen. Die heiße flüssige HPN-Schmelze wird dabei im Vorkühler (2) auf eine Temperatur in der Nähe des Schmelzpunktes (50°C) abgekühlt. Im nachfolgenden Vorkristallisator (3) erfolgt eine teilweise Kristallisation durch weitere Wärmeabfuhr aus der Schmelze. Dabei wird über die Höhe der abgeführten Wärmemenge der gewünschte Kristallgehalt in der Schmelze eingestellt. Die so aus in der Schmelze dispergierten Kristallen gebildete Kristallmaische ist ein 2-Phasen-Gemisch. Durch teilweise Rückführung der Kristallmaische an den Eintritt des Vorkristallisators (3) mittels einer Pumpe wird ein erhöhter Homogenitätsgrad erreicht. Vorzugsweise ist dabei die Menge der durch den Vorkristallisator fließenden Schmelze beziehungsweise Maische wesentlich größer gegenüber der zum Tropfenformer geförderten Menge. Das Verhältnis der Kristallkeime in der zu tropfenden Schmelze in bezug auf die flüssigen Bestandteile bestimmt die Tropfenform und das Verfestigungsverhalten der Tropfen auf dem Kühlband. Durch Verwendung einer Schmelze mit einem hohen Kristallgehalt ist dabei die Bildung von sphärischen Granulatkörpern möglich. Ein geeigneter Vorkristallisator ist in der DE-C-32 09 747 beschrieben.

Zur Herstellung der tropfenförmigen HPN-Granulate wird die Kristallschmelze beziehungsweise Kristallmaische mit einer Pumpe zum Tropfenformer gefördert. Mit Hilfe des Tropfenformers wird die kristallhaltige Schmelze auf das Kühlband getropft, auf dem die Erstarrung der Tropfen erfolgt. Ein geeigneter Tropfenformer ist beispielsweise in der DE-C-32 09 747 beschrieben.

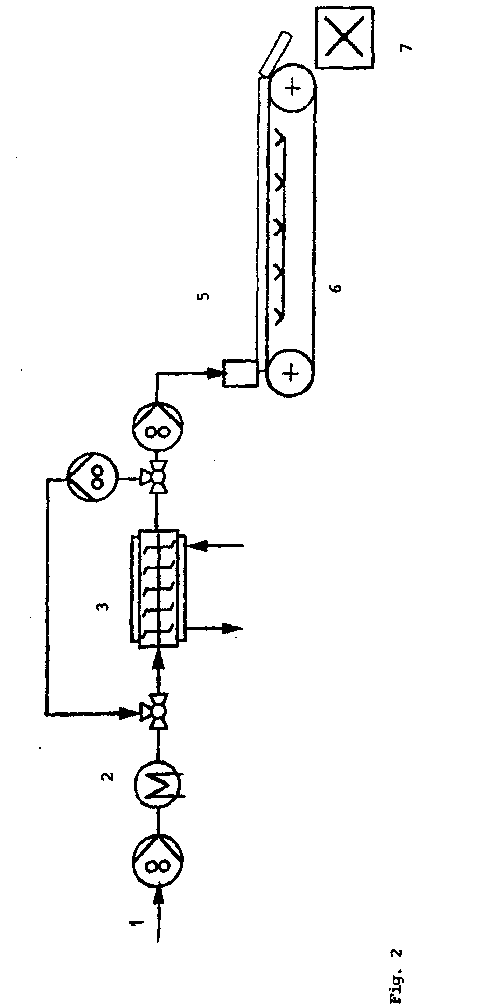

In einer weiteren Ausführungsform der Erfindung, die in Figur 2 dargestellt ist, wird anstelle des Tropfenformers (4) ein Flächenaufgabesystem (5) verwendet. Die Herstellung der kristallhaltigen Schmelze erfolgt wie beim vorstehend beschriebenen Verfahren. Das Flächenaufgabesystem (5) ist so gestaltet, daß die Schmelze über im wesentlichen die gesamte Breite des Kühlbandes (6) verteilt wird. Hierzu kann das Kühlband mit einer Randbegrenzung versehen sein, um über die gesamte Breite des Bandes eine gleichmäßige Schichtdicke zu erzielen. Die Schichtdicke der so gebildeten flächigen Schicht beträgt vorzugsweise mindestens 15 mm, besonders bevorzugt 15 bis 30 mm. Durch Verwendung der kristallhaltigen Schmelze, die auf dem Kühlband (6) schnell erstarrt, ist es möglich, die vorgenannten hohen Schichtdicken zu erreichen. Am Bandende des im Kreis geführten Bandes bricht die erstarrte Schicht beispielsweise aufgrund der Schwerkraft ab und wird in einem Brecher (7) weiter zerkleinert. Hierbei werden flächige Granulate mit einem mittleren Teilchendurchmesser von 20 bis 100 mm, vorzugsweise 20 bis 50 mm, insbesondere 20 bis 35 mm und mindestens 2 im wesentlichen parallelen Flächen, die einen Abstand von vorzugsweise 15 bis 30 mm aufweisen, erhalten. Diese HPN-Granulate sind vorzugsweise quaderähnlich beziehungsweise isometrisch geformt. Die erhaltenen HPN-Granulate zeigen eine sehr geringe Neigung zum Verbacken.

Die Erfindung betrifft weiterhin die Verwendung einer Granuliervorrichtung für eine Schmelze, umfassend nacheinander über Zuleitungen verbunden einen Vorkühler (2), einen Vorkristallisator (3), einen Tropfenformer (4) oder ein Flächenaufgabesystem (5) zum Aufbringen der Schmelze auf ein nachfolgendes Kühlband (6), an das sich bei Verwendung eines Flächenaufgabesystems (5) ein Brecher (7) zur Granulierung von Hydroxipivalinsäureneopentylglykolester anschließt.

Die erfindungsgemäßen Granulate weisen eine höhere Schüttdichte und bessere Dosierbarkeit auf als bekannte Granulate. Insbesondere die im wesentlichen sphärischen Granulate sind leicht dosierbar und haben eine sehr hohe Schüttdichte.

## Patentansprüche

1. Verfahren zur Herstellung von Hydroxipivalinsäureneopentylglykolester-Granulaten durch Aufbringen einer Hydroxipivalinsäureneopentylglykolester-Schmelze auf eine Kühlfläche, auf der die Schmelze erstarrt, **dadurch gekennzeichnet, daß** die Schmelze 15 bis 55 Gew-%, bezogen auf die Gesamtmenge an Hydroxipivalinsäureneopentylglykolester, an Hydroxipivalinsäureneopentylglykolester-Kristallen enthält, die durch Führen einer Hydroxipivalinsäureneopentylglykolester-Schmelze durch einen Vorkristallisator erzeugt werden, wobei die Hydroxipivalinsäureneopentylglykolester-Schmelze zunächst durch einen Vorkühler und dann durch den Vorkristallisator geführt wird, hinter dem ein Teil der Schmelze abgezweigt und in den Vorkristallisator zurückgeführt wird, und der verbleibende Teil der Schmelze über einen Tropfenformer oder ein Flächenaufgabesystem auf ein kontinuierlich angetriebenes Kühlband aufgebracht wird und auf diesem erstarrt und bei Verwendung eines Flächenaufgabesystems nach Verlassen des Kühlbandes in einem Brecher in Granulatform gebracht wird.

2. Im wesentlichen sphärisches Hydroxipivalinsäureneopentylglykolester-Granulat mit einem mittleren Teilchendurchmesser von 3 bis 16 mm.

3. Hydroxipivalinsäureneopentylglykolester-Granulat mit einem mittleren Teilchendurchmesser von 20 bis 100 mm und mindestens zwei im wesentlichen parallelen Flächen, die einen Abstand von 15 bis 30 mm aufweisen.

4. Verwendung einer Granuliervorrichtung für eine Schmelze, umfassend nacheinander über Zuleitungen verbunden einen Vorkühler (2), einen Vorkristallisator (3), einen Tropfenformer (4) oder ein Flächenaufgabesystem (5) zum Aufbringen der Schmelze auf ein nachfolgendes Kühlband (6), an das sich bei Verwendung eines Flächenaufgabesystems (5) ein Brecher (7) anschließt, zur Granulierung von Hydroxipivalinsäureneopentylglykolester.

## Claims

1. A method for preparing neopentyl glycol hydroxypivalate granules by application of a neopentyl glycol hydroxypivalate melt onto a cooling surface on which the melt solidifies, **characterized in that** the melt contains 15 to 55 wt% of neopentyl glycol hydroxypivalate crystals, based on the total amount of neopentyl glycol hydroxypivalate, which are generated by passing a neopentyl glycol hydroxypivalate melt through a precrystallizer, wherein the neopentyl glycol hydroxypivalate melt is initially passed through a precooler and then through the precrystallizer, downstream of which a portion of the melt is shunted off and recycled into the precrystallizer and the remaining portion of the melt is applied, via a droplet former or an areal application system, to a continuously driven cooling belt, and solidifies thereon and, if an areal application system is used, having left the cooling belt is converted to granules in a crusher.

2. Essentially spherical neopentyl glycol hydroxypivalate granules having an average particle diameter of from 3 to 16 mm.

3. Neopentyl glycol hydroxypivalate granules having an average particle diameter of from 20 to 100 mm and at least two essentially parallel faces which are spaced at from 15 to 30 mm.

4. The use of a granulating apparatus for a melt, which comprises, successively connected via lines, a precooler (2), a precrystallizer (3), a droplet former (4) or an areal application system (5) for applying the melt to a downstream cooling belt (6) which, if an areal application system (5) is used, is followed by a crusher (7), for granulating neopentyl glycol hydroxypivalate.

## Revendications

1. Procédé de préparation de granulats de l'ester néopentylglycol de l'acide hydroxypivalique, au moyen de l'application d'une matière à l'état fondu constituée de l'ester néopentylglycol de l'acide hydroxypivalique sur une surface de refroidissement, sur laquelle la matière à l'état fondu se solidifie, **caractérisé en ce que** la matière à l'état fondu contient des cristaux de l'ester néopentylglycol de l'acide hydroxypivalique à raison de 15% à 55% en poids, par rapport à la quantité totale de l'ester néopentylglycol de l'acide hydroxypivalique, cristaux que l'on obtient en conduisant une matière à l'état fondu constituée de l'ester néopentylglycol de l'acide hydroxypivalique au travers d'un précristallisateur, procédé dans lequel on conduit la matière à l'état fondu d'ester néopentylglycol de l'acide hydroxypivalique dans un premier temps au travers d'un prérefroidisseur et par la suite au travers du précristallisateur, on sépare, à la sortie de ce dernier, une partie de la matière à l'état fondu, afin de la recycler dans le précristallisateur, et on dépose, au moyen d'un dispositif formant des gouttes ou au moyen d'un système d'alimentation en nappe, la partie restante de la matière à l'état fondu sur un convoyeur de refroidissement continu, sur lequel elle se solidifie et, dans le cas où l'on met en oeuvre un système d'alimentation en nappe, on procède, après la sortie du convoyeur de refroidissement, à une granulation dans un broyeur.

2. Les granulats d'ester néopentylglycol de l'acide hydroxypivalique présentent une forme essentiellement sphérique et une granulométrie comprise entre 3 mm et 16 mm.

3. Les granulats d'ester néopentylglycol de l'acide hydroxypivalique présentent une granulométrie comprise entre 20 mm et 100 mm, et au moins deux surfaces qui sont pour l'essentiel parallèles et qui présentent un espacement compris entre 15 mm et 30 mm.

4. Mise en oeuvre d'un dispositif de granulation pour les matières à l'état fondu qui comprend successivement, reliés par des canalisations d'alimentation, un prérefroidisseur (2), un précristallisateur (3), un dispositif de formation des gouttes (4) ou un système d'alimentation en nappe (5) pour déposer par la suite la matière à l'état fondu sur un convoyeur de refroidissement (6), et en suivant, dans le cas où l'on a employé le système d'alimentation en nappe (5), un broyeur (7) afin de granuler l'ester néopentylglycol de l'acide hydroxypivalique.
